# EUROPEAN PATENT APPLICATION

(11) **EP 4 226 916 A1**
(43) Date of publication of application: **16.08.2023**
(21) Application number: 22156898.3
(22) Date of filing: 15.02.2022
(51) Int. Cl.: A61K 9/127, A61K 9/51, A61K 31/7052

(54) **LIPID NANOPARTICLE COMPRISING PHOSPHATIDYLSERINE**

(71) Applicant: Universität Basel, 4001 Basel (CH)
(72) Inventor: The designation of the inventor has not yet been filed
(74) Representative: Vossius & Partner Patentanwälte Rechtsanwälte mbB

(57) **Abstract**

The invention relates to a lipid nanoparticle (LNP) comprising at least one cationic matrix-forming lipid and 0.5 mol% to 10 mol% phosphatidylserine, helper lipids and/or therapeutic agents such as proteins, negatively charged nanoparticles, nucleic acids. The invention further relates to a vector, pharmaceutical composition and/or needle-free injection device comprising the LNP of the invention. The invention also provides methods for production of the LNP and vector of the invention.

## Description

The invention relates to a lipid nanoparticle (LNP) comprising at least one cationic matrix-forming lipid and 0.5 mol% to 10 mol% phosphatidylserine, helper lipids and/or therapeutic agents such as proteins, negatively charged nanoparticles, nucleic acids. The invention further relates to a vector, pharmaceutical composition and/or needle-free injection device comprising the LNP of the invention. The invention also provides methods for the production of the LNP and vector of the invention.

LNP formulations can be composed of charged lipids and lipid-like adjuvants that bind and condense negatively charged nucleic acids e.g. through the aid of "helper lipids" into a single nanoparticle. The first generation of LNPs uses permanently positively charged lipids (Feigner, P. L. et al., 1987, Proc Natl Acad Sci U S A 84, 7413-7417; Hafez, I. M., Maurer, N. & Cullis, P. R., 2001, Gene Therapy 8, 1188-1196). This pioneering cationic LNP type is effective in in vitro studies but often fails when applied in vivo. The permanent cationic nature brands the first generation LNPs with high systemic toxicity and poor pharmacokinetic properties(Stewart, M. J. et al., 1992 Human Gene Therapy 3, 267-275; San, H. et al., 1993, Human Gene Therapy 4, 781-788 ). Due to this, clinically translated LNP therapies are based on ionizable lipids. Ionizable LNPs have been developed to circumvent the permanent cationic charge and associated side effects. The chemical nature of ionizable lipids with a pKa - 6.7 (i.e. DODMA and DlinMC3) can be leveraged to formulate pH-responsive nucleic acid baring LNPs. Most ionizable LNP formulations are targeted to the liver through endogenous surface binding of apolipoprotein E (ApoE) and recognition by the LDL receptor. As a direct result of their charge, second-generation ionizable LNPs are more potent and less toxic than their positively charged precursors. To improve the delivery efficiency extensive work has been conducted by academic and biotech industry R&D to further improve the potency of ionizable lipids. However, a spotlight on improving LNPs effectiveness through alteration of the chemical structure of the ionizable lipid components has led to a vacuum of studies looking at the role of helper lipids.

Viral membranes comprise the bioactive lipid phosphatidylserine (PS) (Morizono, K. & Chen, I. S. Y., 2014, J Virol 88, 4275-4290 ). By inclusion of PS in their membranes, virions camouflage themselves as dead cells in an act referred to as apoptotic cell mimicry (Amara, A. & Mercer, J., 2015, Nature Reviews Microbiology 13, 461-469). PS acts as an essential co-factor during the infection process of several viral types (Brunton, B. et al., 2019, PLoS Negl Trop Dis 13; Chu, L.-W. et al., 2019, Int J Mol Sci 20; Wang, J., et al., 2017, Journal of virology, 91(2)). Examples, where the infectivity of virions depends on the PS signal, include the EBOLA virus, Epstein Barr Virus, and Hepatitis B virus. In mammalian cells, PS is an important and evolutionarily well preserved "eat me" signal (Doran, A. C., et al., 2019 Nature Reviews Immunology 1-14). During homeostasis, PS is actively kept on the inner leaflet of the cellular membrane by the flippase enzyme family. Only through the induction of apoptosis or necrosis PS gets gradually exposed to the outer leaflet of the cell membrane and marks the cell with an "eat me" signal. Due to the constant turnover of cells, the process of PS recognition and apoptotic cell clearance (efferocytosis) is highly evolutionarily conserved. Efferocytosis is performed by professional phagocytes, such as macrophages, and 'non-professional' phagocytes, such as epithelial cells, which greatly outnumber the former (Davies, S. P. et al., 2018, Front Immunol 9).

To this day, despite all efforts, only a few LNP products reach late-stage clinical development.

Thus, there is a need to increase the effectiveness of LNP-delivery properties.

The above technical problem is solved by the embodiments disclosed herein and as defined in the claims.

Accordingly, the invention relates to, inter alia, the following embodiments:
1. A lipid nanoparticle (LNP) comprising at least one cationic matrix-forming lipid and 0.5 mol% to 10 mol% phosphatidylserine (PS).
2. The LNP of embodiment 1 additionally comprising a protein and/or a negatively charged nanoparticle.
3. A vector comprising the LNP of embodiment 1 or 2 and a nucleic acid.
4. The vector of embodiment 3, wherein the nucleic acid is condensed nucleic acid.
5. The vector of embodiment 3 or 4, wherein the vector has an N/P ratio between 1 and 10.5.
6. The LNP of any one of embodiments 1 or 2 or the vector of any one of embodiments 3 to 5, wherein the LNP additionally comprises at least one helper lipid.
7. The LNP of embodiment 6, or the vector of embodiment 6, wherein the helper lipid comprises a PEG-modified lipid, preferably accounting for 0.5 mol% to 10 mol% of the LNP mass.
8. The LNP of embodiment 6 or 7, or the vector of embodiment 6 or 7, wherein the helper lipid comprises DOPC and/or cholesterol.
9. A pharmaceutical composition comprising the LNP of any one of embodiments 1, 2 or 6 to 8, or the vector of any one of embodiments 3 to 8 and a pharmaceutical carrier.
10. The LNP, vector or pharmaceutical composition of any of the preceding embodiments for use in medicine.
11. The LNP of embodiments 1, 2, 6 to 8, the vector of embodiments 3 to 8 or the pharmaceutical composition of embodiment 9, for use in the treatment and/or prevention of a disease or disorder selected from the group of genetic disease or disorder, infectious disease or disorder, cancerous disease or disorder.
12. The LNP, vector or pharmaceutical composition for use of embodiment 11, wherein the infectious disease or disorder is a viral infection.
13. The LNP, vector or pharmaceutical composition for use of embodiment 11, wherein the genetic disease or disorder is sickle cell anemia.
14. The LNP, vector or pharmaceutical composition for use of embodiment 11, genetic disease or disorder is amyloidosis.
15. A needle-free injector device comprising the pharmaceutical composition of embodiment 9, the LNP of any one of embodiments 1, 2 or 6 to 8, or the vector of any one of embodiments 3 to 8.
16. A method for producing a vector of any of the embodiments 3 to 8 comprising the steps of:
   a) mixing a matrix-forming lipid, an ionizable lipid with 0.5 mol% to 10 mol% phosphatidylserine;
   b) obtaining a lipid nanoparticle by facilitated self-assembly and by a size-dependent separation method of the mixture of step (a); and
   c) obtaining the vector by loading the lipid nanoparticle with a nucleic acid.
17. A method for producing a vector of any one of the embodiments 3 to 8 comprising the steps of:
   a) mixing a matrix-forming lipid, an ionizable lipid and 0.5 mol% to 10 mol% phosphatidylserine with a nucleic acid; and
   b) obtaining the vector by facilitated self-assembly and by a size-dependent separation method of the mixture of step (a).

The term "lipid nanoparticle" or "LNP", as used herein, refers to a compact nanoparticle at least partially composed of lipids that may be used as a pharmaceutical delivery agent. A nanoparticle is typically spherical with an average diameter between 10 and 1000 nanometers. The lipid nanoparticle described herein is compact in that it comprises a core matrix that it is not "hollow" or comprises less water-filled space than, e.g., a liposome, preferably a non-aqueous core. Lipid nanoparticles can be distinguished from Liposomes and/or Micelles by methods known in the art, e.g, by Cryo-TEM, SEM, X-ray, dynamic light scattering, sedimentation field flow fractionation, optical microscopy and/or zeta potential measurement (see e.g. Hallan, S. S. et al., 2021, Pharmaceutics, 13(4), 549). In some embodiments, the lipid nanoparticle described herein has an envelope structure such as one or more lipid mono- or bilayers surrounding the core matrix. In some embodiments, the lipid nanoparticle described herein has a micellar-like structure. In some embodiments, the lipid nanoparticle comprises at least 50 mol%, 60 mol%, 70 mol%, 80 mol% or 90 mol% lipids.

The term "cationic matrix-forming lipid", as used herein, refers to a lipid that is able to form a lipid nanoparticle core matrix and is cationic or becomes cationic (protonated) as the pH is lowered below the pK of the ionizable group of the lipid, but is progressively more neutral at higher pH values. At pH values below the pK, the cationic matrix-forming lipid is then able to associate with negatively charged nucleic acids (e.g., oligonucleotides). As used herein, the term "cationic matrix-forming lipid" includes zwitterionic lipids that assume a positive charge on pH decrease.

In some embodiments the cationic matrix-forming lipid described herein comprises a lipid species which carry a net positive charge at a selective pH, such as physiological pH. Such lipids include, but are not limited to, N,N-dioleyl-N,N-dimethylammonium chloride (DODAC); N-(2,3- dioleyloxy)propyl)-N,N,N-trimethylammonium chloride (DOTMA); N,N-distearyl-N,N- dimethylammonium bromide (DDAB); N-(2,3-dioleoyloxy)propyl)-N,N,N- trimethylammonium chloride (DOTAP); 3-(N-(N',N'-dimethylaminoethane)- carbamoyl)cholesterol (DC-Choi) and N-(l,2-dimyristyloxyprop-3-yl)-N,N-dimethyl-N- hydroxyethyl ammonium bromide (DMRIE). The following lipids are cationic and have a positive charge at below physiological pH: DODAP, DODMA, DMDMA, 1, 2-dilinoleyloxy-N,N- dimethylaminopropane (DLinDMA), 1, 2-dilinolenyloxy-N,N-dimethylaminopropane (DLenDMA).

In one embodiment, the cationic matrix-forming lipid is an amino lipid. Suitable amino lipids useful in the invention include those described in WO 2009/096558, incorporated herein by reference in its entirety. Representative amino lipids include 1, 2-dilinoleyoxy-3- (dimethylamino)acetoxypropane (DLin-DAC), 1, 2-dilinoleyoxy-3-morpholinopropane (DLin-MA), 1, 2-dilinoleoyl-3-dimethylaminopropane (DLinDAP), 1, 2-dilinoleylthio-3- dimethylaminopropane (DLin-S-DMA), 1-linoleoyl-2-linoleyloxy-3-dimethylaminopropane (DLin-2-DMAP), 1, 2-dilinoleyloxy-3-trimethylaminopropane chloride salt (DLin-TMA-O),1, 2-dilinoleoyl-3-trimethylaminopropane chloride salt (DLin-TAP CI), 1, 2-dilinoleyloxy-3- (N-methylpiperazino)propane (DLin-MPZ), 3 -(N,N-dilinoley lamino)-1, 2-propanediol (DLinAP), 3-(N,N-dioleylamino)-l, 2-propanedio (DOAP), 1, 2-dilinoleyloxo-3-(2-N,N- dimethylamino)ethoxypropane (DLin-EG-DMA), and 2,2-dilinoleyl-4- dimethylaminomethy 1- [1, 3] -dioxolane (DLin-K-DM A).

In some embodiments, the concentration of cationic matrix-forming lipids in the LNPs described herein is from 20 to about 80 mol %, preferably from 30 to about 75 mol %, more preferably from about 40 to about 75 mol %, more preferably from about 40 to about 60 mol %, more preferred about 50 mol % based on the total number of mol of the LNP.

The terms "phosphatidylserine" and "PS" are used herein interchangeably and refer to a phosphoglyceride having a phosphorylserine head group. In some embodiments, the phosphatidylserine described herein has the following structure:

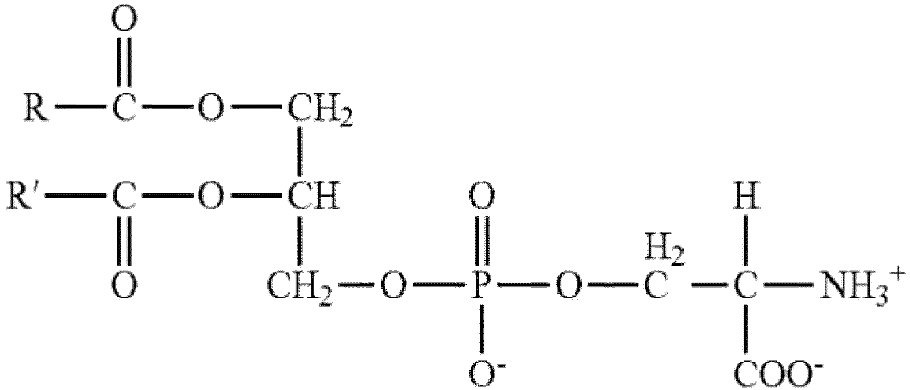

The R and R' moieties are fatty acids moieties, typically naturally occurring fatty acids or derivatives of naturally occurring fatty acids. In some embodiments, the fatty acid moieties described herein are saturated or unsaturated fatty acid moieties. In some embodiments, the fatty acid moieties described herein have at least 8 carbon atoms. In some embodiments, the fatty acid moieties have between 8 and 26, between 8 and 21, between 24 and 26 orbetween 14 and 21 carbon atoms. In some embodiments, the fatty acid moieties have at least 21 carbon atoms. In some embodiments, the fatty acid moieties have about 18 carbon atoms. In some embodiments R and R' are the same fatty acid moieties. In some embodiment, the fatty acid moieties are both myristoyl. In another embodiment, the fatty acid moieties are both palmitoyl. In another embodiment, the fatty acid moieties are both stearoyl. In another embodiment, the fatty acid moieties are both arachidoyl. In another embodiment, the fatty acid moieties are myristoyl and stearoyl. In another embodiment, the at least one, preferably borth fatty acid moieties are selected from the group consisting of arachidoyl, palmitoyl, myristoyl and stearoyl.

The content of PS in the LNPs described herein is from about 0.5 to about 10 mol % and depends on factors such as type of PS, the desired size of the LNP, the desired Zeta distribution, agents to be delivered, delivery site, particle stability, systemic circulation time, delivery amount and other LNP ingredients. In some embodiments, the concentration of PS in the LNPs described herein is from about 0.5 to about 10 mol %, or from about 0.5 to about 7.5 mol %, or from about 0.5 to about 5 mol %, or from about 1 to about 10 mol %, or from about 1 to about 7.5 mol %, or from about 1 to about 5.5 mol %, or from about 1.5 to about 10 mol %, or from about 1.5 to about 7.5 mol %, or from about 1.5 to about 5.5 mol %, or from about 2 to about 10 mol %, or from about 2 to about 7.5 mol %, or from about 2 to about 5.5 mol %, or from about 2.5 to about 10 mol %, or from about 2.5 to about 7.5 mol %, or from about 2.5 to about 5.5 mol %.

The incorporation of negatively charged PS in LNPs appears counterintuitive since many agents to be delivered have a negative charge.

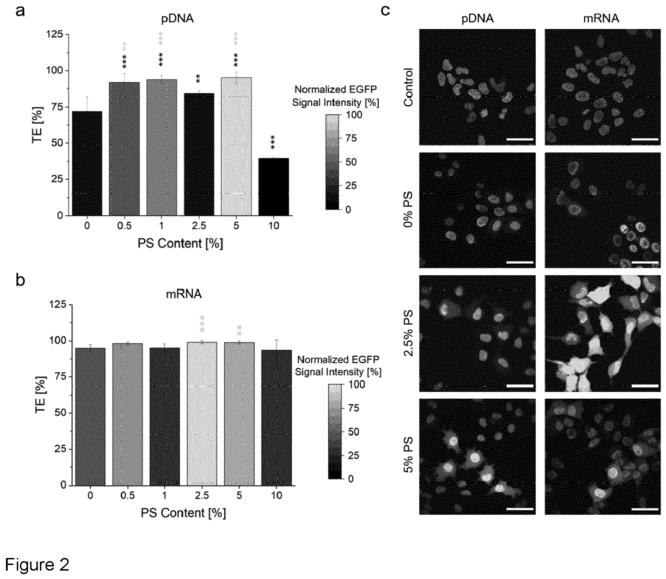

However, the inventors found that PS embodies an "eat me" signal for cells and therefore improves cell uptake and delivery (Fig. 2, 3, 4; Example 5, 6, 7, 8). Further, the inventors found that LNPs with improved physical properties (Tables 1 and 2; Example 1, 2, 3, 4) and masking of positive charge by the surface presentation of PS (Example 4). The effect can be observed in various formulations (Fig. 2, 3, 4).

The LNP described herein is suitable for the delivery of any agent, particularly negatively charged agents. In some embodiments, the LNP described herein comprises a nucleic acid (e.g. a DNA or RNA based vaccine), a protein (e.g. gene therapy), a small molecule (e.g. a vitamin, antibiotics, cosmetics), negatively charged nanoparticle (e.g. a diagnostic marker, or an isotope).

Accordingly, the invention is at least in part based on the surprising finding that phosphatidylserine can improve the delivery properties of LNPs.

In certain embodiments, the invention relates to the LNP of the invention additionally comprising a protein and/or a negatively charged nanoparticle.

In some embodiments, the protein described herein is a negatively charged protein or a protein composition with an overall negative charge. In some embodiments, the protein described herein has a weight of less than about 500 kDa, less than about 400 kDa, less than about 350 kDa, less than about 300 kDa, or less than about 250 kDa. In some embodiments, the protein described herein is a protein of a gene-editing method, preferably a CRISPR-associated protein.

The term "negatively charged nanoparticle", as used herein, refers to any particle of a size less than 300 nm, typically between 20 and 300nm preferably 100 nm or less, and most preferably about 20-50 nm, that has an overall negative charge. In some embodiments, the negatively charged nanoparticle described herein comprises at least one agent selected from the group of imaging probe, radioisotope, or a metal. In some embodiments, the negatively charged nanoparticle described herein comprises gold.

The protein content and/or negatively charged nanoparticle content in the LNPs described herein is typically from about 10 to about 90 mol % and depends on factors such as type of PS, type of additional ingredients, the desired size of the LNP, the desired Zeta distribution, agents to be delivered, delivery site, particle stability, systemic circulation time, delivery amount and other LNP ingredients.

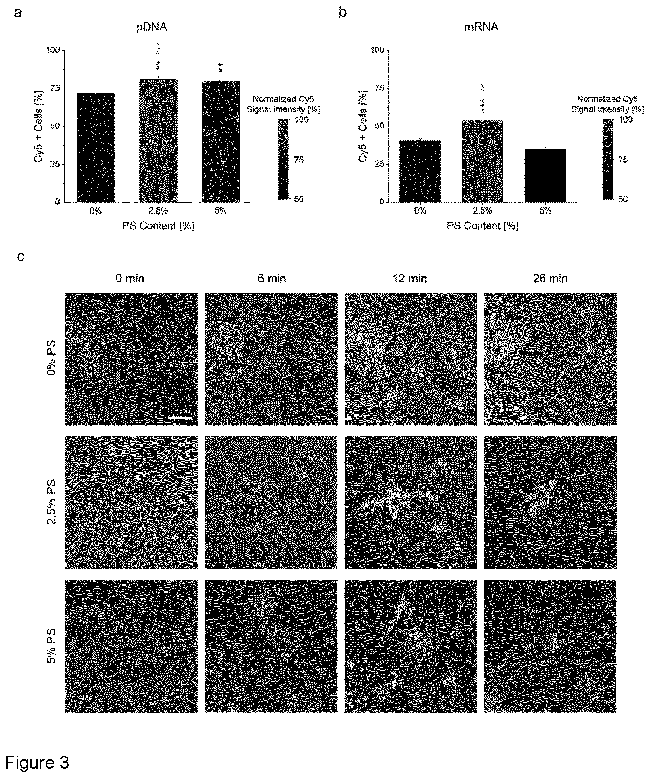

The improved cell uptake and delivery (Fig. 3; Example 7) of the LNP of the invention are particularly useful for the delivery of proteins and/or negatively charged nanoparticles that are otherwise difficult to deliver.

Accordingly, the invention is at least in part based on the surprising finding that phosphatidylserine can improve the delivery of proteins and/or negatively charged nanoparticles with LNPs.

In certain embodiments, the invention relates to a vector comprising the LNP of the invention and a nucleic acid.

The term "vector", as used herein, refers to an agent, capable of transferring or transporting another nucleic acid molecule. The transferred nucleic acid is generally linked to or inserted into the vector agent. A vector may include sequences that direct autonomous replication in a cell or may include sequences sufficient to allow integration into host cell DNA.

The term "nucleic acid", as used herein, refers to a molecule composed of at least two monomelic nucleotides, and can include, for example, ribonucleic acids (e.g., premRNA, mRNA, siRNA or rRNA molecules), deoxyribonucleic acids (e.g., DNA, cDNA or gDNA), single-stranded nucleic acids, double-stranded nucleic acids, naturally occurring nucleotides, analogs of naturally occurring nucleotides, or a combination thereof. In some embodiments, the nucleic acid described herein is selected from the group of siRNA, mRNA and DNA.

The content of nucleic acid in the LNPs described herein is from about 10 to about 90 mol % and depends on factors such as type of PS, the desired size of the LNP, the desired Zeta distribution, agents to be delivered, delivery site, particle stability, systemic circulation time, delivery amount and other LNP ingredients.

The improved cell uptake and delivery (Fig. 3; Example 7) of the LNP of the invention are particularly useful for the delivery of nucleic acids, wherein an efficient and complete delivery is particularly important.

Accordingly, the invention is at least in part based on the surprising finding that phosphatidylserine can improve the transfection efficacy of nucleic acids with LNPs.

In certain embodiments, the invention relates to the vector of the invention, wherein the vector has an N/P ratio between about 1 and about 12, preferably between 1 and about 10.5.

The term "N/P ratio", as used herein, refers to the molar ratio of ionizable (in the physiological pH range) nitrogen atoms in a lipid to phosphate groups in a nucleic acid, e.g., in an LNP to a nucleic acid.

The negative charge can influence the surface charge of LNPs and plays into the N/P ratio between the nucleic acid condensing lipid and nucleic acids (Tsui, F. C., et al., 1986, Biophysical Journal 49, 459-468 (1986); Bailey, A. L. & Cullis, P. R., 1997, Biochemistry 36, 1628-1634).

Without being bound by theory, the negative charge of PS directly appears to play into the N/P ratio and can therefore be compensated by a higher amount of positive charge. Therefore, the LNPs of the invention require different N/P ratios (Tables 1 and 2; Fig. 5, 6, 7, 8) than LNPs not comprising PS.

In certain embodiments, the invention relates to the LNP of the invention, or the vector of the invention, wherein the LNP additionally comprises at least one helper lipid.

The term "helper lipid", as used herein, refers to lipids other than the cationic matrix-forming lipid, that can influence the properties of the LNP. In some embodiments, the helper lipid described herein is selected from the group of cone-shape geometry favoring helper lipid (e.g. dioleoylphosphatidylethanolamine (DOPE)), cylindrical-shaped helper lipid (e.g. phosphatidylcholine), Cholesterol, and (pH-sensitive) anionic helper lipids (e.g. fatty acids and cholesteryl hemisuccinate (CHEMS)). The person skilled in the art is aware of how to choose the appropriate content of the helper lipids (see e.g., Cheng, X., & Lee, R. J., 2016, Advanced drug delivery reviews, 99, 129-137).

The content of helper lipid in the LNPs described herein is typically from about 0.001 to about 50 mol % and depends on factors such as type of helper lipid, type of PS, type of additional ingredients, the desired size of the LNP, the desired Zeta distribution, agents to be delivered, delivery site, particle stability, systemic circulation time, delivery amount and other LNP ingredients.

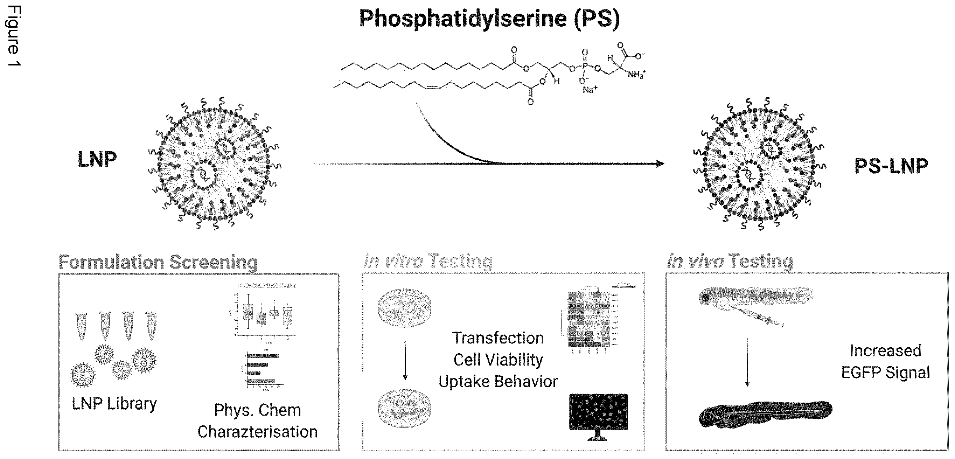

The inventors found that helper lipids play an important role in the effectiveness of PS-NLP (Tables 1 and 2; Figures 1,2,3,4).

In certain embodiments, the invention relates to the LNP of the invention, or the vector of the invention, wherein the helper lipid comprises a PEG-modified lipid, preferably accounting for about 0.5 to about 10 mol %, and depends on factors such as type of PEG-modified lipid, type of other helper lipids, type of PS, type of additional ingredients, the desired size of the LNP, the desired Zeta distribution, agents to be delivered, delivery site, particle stability, systemic circulation time, delivery amount and other LNP ingredients.

The term "PEG-modified lipid", as used herein, refers to a lipid-linked (e.g. covalently attached) to at least one PEG polymer chain. In some embodiments, the PEG-modified lipid described herein comprises a poly(ethylene) glycol chain of up to 5, 10 or 20 kDa in length covalently attached to a lipid with alkyl chain(s) of C6-C20 length. In some embodiments, the PEG-modified lipid is reversibly linked to the LNP described herein and the PEG moiety is gradually released in blood circulation upon administration.

In some embodiments, the PEG-modified lipid described herein is a PEG-phospholipid or a PEG-ceramide. In some embodiments, the PEG-modified lipid is a PEG-ceramide selected from the group of PEG2000-DSPE, PEG2000-DPPE, PEG2000-DMPE, PEG2000-DOPE, PEG1000-DSPE, PEG1000-DPPE, PEG1000-DMPE, PEG1000-DOPE, PEGS 50-DSPE, PEG550-DPPE, PEG-550DMPE, PEG-1000DOPE, PEG-BML, PEG-Cholesterol. PEG2000-Ceramide, PEG1000-Ceramide, PEG750-Ceramide and PEG550-Ceramide.

The inventors found that the inclusion of a PEG-modified lipid can enhance LNP colloidal stability and circulation time of the LNPs of the invention without substantially reducing uptake or inhibiting endosomal release (Example 17).

Accordingly, the invention is at least in part based on the surprising finding that PEG-modified lipids can improve the stability of LNPs comprising phosphatidylserine.

In certain embodiments, the invention relates to the LNP of the invention, or the vector of the invention, wherein the helper lipid comprises DOPC and/or cholesterol.

The content of DOPC and/or cholesterol in the LNPs described herein is from about 20 to about 80 mol % and depends on factors such as type of DOPC and/or cholesterol, type of other helper lipids, type of PS, type of additional ingredients, the desired size of the LNP, the desired Zeta distribution, agents to be delivered, delivery site, particle stability, systemic circulation time, delivery amount and other LNP ingredients.

The inventors found that DOPC and cholesterol play an important role in the effectiveness of PS-NLP.

Accordingly, the invention is at least in part based on the surprising finding that DOPC and/or cholesterol can alter and/or improve the phosphatidylserine-mediated improvement of delivery properties of LNPs.

In certain embodiments, the invention relates to a pharmaceutical composition comprising the LNP of the invention, or the vector of the invention and a pharmaceutical carrier.

The term "pharmaceutical carrier", as used herein, refers to an ingredient in the composition, other than the active ingredient(s), which is nontoxic to recipients at the dosages and concentrations employed. A pharmaceutical carrier includes, but is not limited to, a buffer, excipient, stabilizer, or preservative.

Pharmaceutical carriers include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

In certain embodiments, the invention relates to the LNP, vector or pharmaceutical composition of the invention for use in medicine.

The phrase "use in medicine", as used herein, also refers to "use in treatment". The term "treatment" (and grammatical variations thereof such as "treat" or "treating"), as used herein, refers to clinical intervention in an attempt to alter the natural course of the individual being treated, and can be performed either for prophylaxis (e.g. vaccination) or during the course of clinical pathology. Desirable effects of treatment include, but are not limited to, preventing occurrence or recurrence of disease, alleviation of symptoms, diminishment of any direct or indirect pathological consequences of the disease, decreasing the rate of disease progression, amelioration or palliation of the disease state, and remission or improved prognosis. In some embodiments, antibodies of the invention are used to delay the development of a disease or to slow the progression of a disease. The term "treatment of a disease or disorder" also includes treatment of a symptom of the respective disease or disorder. In some embodiments, the treatment described herein also includes "prevention". The term "prevention", as used herein, relates to the capacity to prevent, minimize or hinder the onset or development of a disorder, disease or condition before its onset.

In some embodiments, the invention relates to the LNP, the vector or the pharmaceutical composition of the invention for use in treatment, wherein the LNP is administered in doses of at least 0.1 µg, at least 0.5 µg, at least 1 µg, at least 10 µg or at least 30 µg in the range from about 0.1 µg to about 1000 µg, about 0.5 µg to about 500 µg, about 0.2 µg to about 200 µg, about 1 µg to about 150 µg, or about 9 µg to about 110 µg to achieve a therapeutic effect.

In some embodiments, the invention relates to the vector or the pharmaceutical composition of the invention for use in treatment, wherein the vector is administered in doses in the range from at least 10⁶, 10⁷, 10⁸, 10⁹, 10¹⁰, 10¹¹, 10¹², 10¹³, 10¹⁴, 10¹⁵, 10¹⁶, or more vector genomes per kilogram (vg/kg) of the weight of the subject, to achieve a therapeutic effect.

Accordingly, the invention is at least in part based on the surprising finding that the LNPs described herein can improve the delivery of therapeutic agents and target site-specific delivery.

In certain embodiments, the invention relates to the LNP, vector or pharmaceutical composition for use the invention, for use in the treatment and/or prevention of a disease or disorder selected from the group of genetic disease or disorder, infectious disease or disorder, cancerous disease or disorder.

The term "genetic disease or disorder", as used herein, refers to any disease or abnormality that results from inherited factors. In some embodiments, the genetic disease or disorder is a disease or disorder selected from the group of Angelman syndrome, Canavan disease, Charcot-Marie-Tooth disease, Color blindness, Cri du chat syndrome, cystic fibrosis, DiGeorge syndrome, Down syndrome, Duchenne muscular dystrophy, familial hypercholesterolemia, haemochromatosis, hemophilia, Klinefelter syndrome, neurofibromatosis, phenylketonuria, polycystic kidney disease, Prader-Willi syndrome, sickle cell disease, spinal muscular atrophy, Tay-Sachs disease and Turner syndrome.

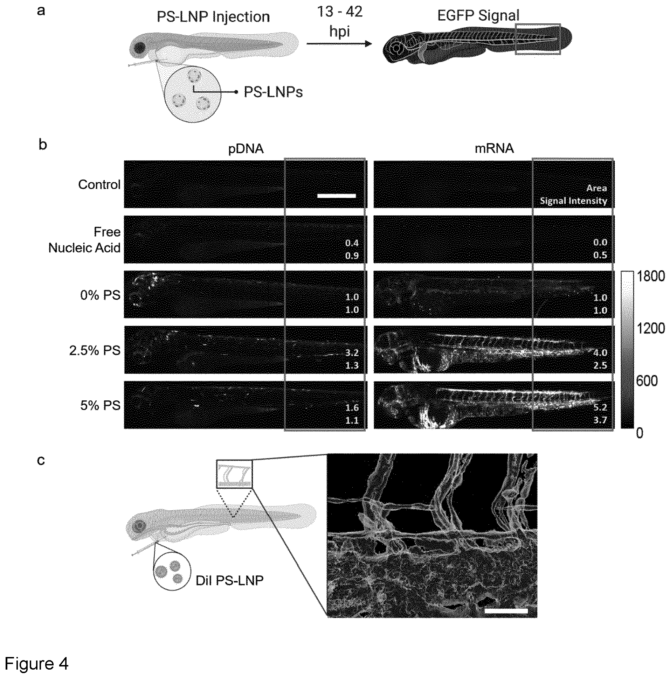

The improved cell uptake and delivery (Fig. 3, 4; Example 6) of the LNPs of the invention are particularly useful for delivering treatment for genetic diseases or disorders, wherein a complete and efficient is particularly important.

Accordingly, the invention is at least in part based on the surprising finding that the LNPs described herein can improve delivery (e.g. transfection efficiency) of therapeutic vectors.

The term "infectious disease or disorder", as used herein, refers to a disease or disorder characterized by the invasion of at least one body tissue of a subject by one or more transmittable pathogens, their multiplication, and the reaction of host tissues to the infectious pathogen and/or the toxins they produce. In some embodiments, the infectious disease or disorder described herein is a primary infection. In some embodiments, the infectious disease or disorder described herein is an opportunistic infection. In some embodiments, the infectious disease or disorder described herein is a secondary infection. In some embodiments, the pathogen causing the infection described herein is a virus, a bacterium, a fungus, or a parasite. In some embodiments, the infection described herein is an infection selected from the group of lower respiratory infections, HIV infection, diarrheal diseases, tuberculosis, and malaria.

The improved cell uptake and delivery (Fig. 3, 4; Example 6) of the LNPs of the invention are useful for example for the efficient delivery of unstable treatment molecules such as mRNA. Therefore, the LNPs of the invention can enable the efficient activation of the immune system.

Accordingly, the invention is at least in part based on the surprising finding that the LNPs described herein can improve the delivery of vaccinations.

The term "cancerous disease or disorder", as used herein, refers to a disease or disorder characterized by uncontrolled cell growth that can result in unregulated growth, lack of differentiation, local tissue invasion, and metastasis. In some embodiments, the cancerous disease or disorder is selected from the group of breast cancer, prostate cancer, ovarian cancer, cervical cancer, skin cancer, pancreatic cancer, colorectal cancer, renal cancer and lung cancer.

Anti-cancer treatments comprise aggressive agents, wherein the effect is limited by the occurrence of side effects. The improved cell uptake and delivery (Fig. 3, 4; Example 6) of the means and methods of the invention can improve the selective cancer cell-specific uptake of therapeutic agents.

Accordingly, the invention is at least in part based on the surprising finding that the LNPs described herein can improve target site-specific delivery.

In certain embodiments, the invention relates to the LNP, vector or pharmaceutical composition for use of the invention, wherein the infectious disease or disorder is a viral infection.

The term "viral infection", as used herein, refers to a disease or disorder characterized by the invasion of at least one body tissue of a subject by one or more virus, virus multiplication, and/or the reaction of host tissues to the virus and/or the toxins the virus induces. In some embodiments, the viral infection described herein is an infection of a virus from the genus selected from the group consisting of Adenoviridae, Anelloviridae, Arenaviridae, Astroviridae, Bunyaviridae, Bunyavirus, Caliciviridae, Coronaviridae, Filoviridae, Flaviviridae, Hepadnaviridae, Herpesviridae, Orthomyxoviridae, Papillomaviridae, Paramyxoviridae, Parvoviridae, Picornaviridae, Pneumoviridae, Polyomaviridae, Poxviridae, Reoviridae, Retroviridae, Rhabdoviridae, Rhabdovirus and Togaviridae.

In some embodiments, the viral infection described herein is an infection of at least one virus selected from the group consisting of: Aichi virus, Australian bat lyssavirus, BK polyomavirus, Banna virus, Barmah forest virus, Bunyamwera virus, Bunyavirus La Crosse, Bunyavirus snowshoe hare, Cercopithecine herpesvirus, Chandipura virus, Chikungunya virus, Cosavirus A, Cowpox virus, Coxsackievirus, Crimean-Congo hemorrhagic fever virus, Dengue virus, Dhori virus, Dugbe virus, Duvenhage virus, Eastern equine encephalitis virus, Ebolavirus, Echovirus, Encephalomyocarditis virus, Epstein-Barr virus, European bat lyssavirus, GB virus C/Hepatitis G virus, Hantaan virus, Hendra virus, Hepatitis A virus, Hepatitis B virus, Hepatitis C virus, Hepatitis E virus, Hepatitis delta virus, Horsepox virus, Human adenovirus, Human astrovirus, Human coronavirus, Human cytomegalovirus, Human enterovirus 68, Human enterovirus 70, Human herpesvirus 1, Human herpesvirus 2, Human herpesvirus 6, Human herpesvirus 7, Human herpesvirus 8, Human immunodeficiency virus, Human papillomavirus 1, Human papillomavirus 2, Human papillomavirus 16, Human papillomavirus 18 ,Human parainfluenza, Human parvovirus B19, Human respiratory syncytial virus, Human rhinovirus, Human SARS coronavirus, Human spumaretrovirus, Human T-lymphotropic virus, Human torovirus, Influenza A virus, Influenza B virus, Influenza C virus, Isfahan virus, JC polyomavirus, Japanese encephalitis virus, Junin arenavirus, KI Polyomavirus, Kunjin virus, Lagos bat virus, Lake Victoria marburgvirus, Langat virus, Lassa virus, Lordsdale virus, Louping ill virus, Lymphocytic choriomeningitis virus, Machupo virus, Mayaro virus, MERS coronavirus, SARS-CoV-2, Measles virus, Mengo encephalomyocarditis virus, Merkel cell polyomavirus, Mokola virus, Molluscum contagiosum virus, Monkeypox virus, Mumps virus, Murray valley encephalitis virus, New York virus, Nipah virus, Norwalk virus, O'nyong-nyong virus, Orf virus, Oropouche virus, Pichinde virus, Poliovirus, Punta toro phlebovirus, Puumala virus, Rabies virus, Rift valley fever virus, Rosavirus A, Ross river virus, Rotavirus A, Rotavirus B, Rotavirus C, Rubella virus, Sagiyama virus, Salivirus A, Sandfly fever sicilian virus, Sapporo virus, SARS coronavirus 2, Semliki forest virus, Seoul virus, Simian foamy virus, Simian virus 5, Sindbis virus, Southampton virus, St. louis encephalitis virus, Tick-borne powassan virus, Torque teno virus, Toscana virus, Uukuniemi virus, Vaccinia virus, Varicella-zoster virus, Variola virus, Venezuelan equine encephalitis virus, Vesicular stomatitis virus, Western equine encephalitis virus, WU polyomavirus, West Nile virus, Yaba monkey tumor virus, Yaba-like disease virus, Yellow fever virus and Zika virus.

The improved cell uptake and delivery (Fig. 3; Example 6) of the LNPs of the invention are useful for example for the efficient delivery of unstable treatment molecules such as mRNA. Therefore, the LNPs of the invention can enable the efficient activation of the immune system e.g. against viral proteins.

Accordingly, the invention is at least in part based on the surprising finding that the LNPs described herein can improve the delivery of vaccination against viruses.

In certain embodiments, the invention relates to the LNP, vector or pharmaceutical composition for use of the invention, wherein the genetic disease or disorder is sickle cell anemia.

The term "sickle cell anemia", as used herein, refers to a genetic disease or disorder caused by an abnormality in a hemoglobin protein, e.g., hemoglobin S (sickle hemoglobin), HbC, HbD, and HbO-Arab. The term sickle cell anemia also includes diseases such as sickle cell-b° thalassemia, hemoglobin SC disease, or sickle cell-b+ thalassemia. Sickle cell anemia can be diagnosed by sequencing the DNA of a patient for the underlying mutation. Red blood cells in sickle cell anemia become disc-shaped, fragile and inflexible, leading to a variety of symptoms of the disease, e.g., joint pain and other bone pain, fatigue, breathlessness, rapid heart rate, delayed growth and puberty, susceptibility to infections, ulcers on the lower legs (in adolescents and adults), jaundice, bone pain, attacks of abdominal pain, and fever.

Major challenges for current gene therapy of sickle cell disease include insufficient transduction efficiency of the target hemopoietic stem cells, inconsistent expression of the transgene, putative aberrant expression near integration sites raising safety issues and lack of long term expression of the transgene exhibiting eventual silencing (see e.g. Papanikolaou, E., & P Anagnou, N., 2010, Current gene therapy, 10(5), 404-412).

The means and methods of the invention, therefore, address the major challenges of sickle cell anemia gene therapy as described herein (see e.g. Fig 2, 4).

Accordingly, the invention is at least in part based on the surprising finding that the LNPs described herein can improve delivery (e.g. transfection efficiency) of therapeutic vectors.

In certain embodiments, the invention relates to the LNP, vector or pharmaceutical composition for use of the invention, genetic disease or disorder is amyloidosis.

The term "amyloidosis", as used herein, refers to a genetic disease or disorder in which abnormal protein deposits in various tissues. These protein deposits damage the tissues and interfere with the function of the involved organ. The abnormal protein deposits are called amyloid, hence the name of this group of diseases.

The means and methods of the invention, enable the delivery of therapeutic agents to target sites, that are not reached by conventional methods, in particular when the LNP is chemically modified to carry a receptor ligand.

Accordingly, the invention is at least in part based on the surprising finding that the LNPs described herein can improve delivery (e.g. transfection efficiency) of therapeutic vectors.

In certain embodiments, the invention relates to a needle-free injector device comprising the pharmaceutical composition of the invention, the LNP of the invention, or the vector of the invention.

The term "needle-free injector device", as used herein, refers to a delivery system that drives an agent to be delivered through the skin using a force that propels the agent through the skin. In some embodiments, the needle-free injector device uses Lorentz force, shock waves, pressure by gas or electrophoresis as the force to propel the agent to be delivered. In some embodiments the needle-free injector device described herein employs a spring system, a laser-powered system or an energy-propelled system. In some embodiments the needle-free injector device described herein is an intradermal injector, an intramuscular injector or a subcutaneous injector. The production and use of needle-free injector devices are known to the person skilled in the art (See e.g., US7776007; Ravi, A. D. et al., 2015, International journal of pharmaceutical investigation, 5(4), 192-199).

The LNP of the invention can densely pack and protect its load. This enables small delivery amounts and high resistance to the forces which propel the LNP and its load through the skin.

Accordingly, the invention is at least in part based on the surprising finding that the LNPs protect and stabilize therapeutic agents.

In certain embodiments, the invention relates to a method for producing a vector of the invention comprising the steps of: a) mixing a matrix-forming lipid, a cationic matrix-forming lipid with 0.5 mol% to 10 mol% phosphatidylserine; b) obtaining a lipid nanoparticle by facilitated self-assembly and by a size-dependent separation method of the mixture of step (a); and c) obtaining the vector by loading the lipid nanoparticle with a nucleic acid.

In certain embodiments, the invention relates to a method for producing a vector of the invention comprising the steps of: a) mixing a matrix-forming lipid, a cationic matrix-forming lipid and 0.5 mol% to 10 mol% phosphatidylserine with a nucleic acid; and b) obtaining the vector by facilitated self-assembly and by a size-dependent separation method of the mixture of step (a).

"a," "an," and "the" are used herein to refer to one or to more than one (i.e., to at least one, or to one or more) of the grammatical object of the article.

"or" should be understood to mean either one, both, or any combination thereof of the alternatives.

"and/or" should be understood to mean either one, or both of the alternatives.

Throughout this specification, unless the context requires otherwise, the words "comprise", "comprises" and "comprising" will be understood to imply the inclusion of a stated step or element or group of steps or elements but not the exclusion of any other step or element or group of steps or elements.

The terms "include" and "comprise" are used synonymously. "preferably" means one option out of a series of options not excluding other options. "e.g." means one example without restriction to the mentioned example. By "consisting of" is meant including, and limited to, whatever follows the phrase "consisting of."

The terms "about" or "approximately", as used herein, refer to "within 20%", more preferably "within 10%", and even more preferably "within 5%", of a given value or range.

Reference throughout this specification to "one embodiment", "an embodiment", "a particular embodiment", "a related embodiment", "a certain embodiment", "an additional embodiment", "some embodiments", "a specific embodiment" or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments. It is also understood that the positive recitation of a feature in one embodiment, serves as a basis for excluding the feature in a particular embodiment.

The invention will be further described in the following examples, which do not limit the scope of the invention described in the claims. Design and evaluation of lipid nanoparticles (LNPs) equipped with phosphatidylserine (PS) was carried out as schematically represented in **Figure 1****.**

### Brief description of Figures and Tables

**Figure 1****: Design and evaluation of lipid nanoparticles (LNPs) equipped with phosphatidylserine (PS).** LNPs were formulated with PS, DOPC, and cholesterol as helper lipids and DOTAP or DODMA as cationic or ionizable nucleic acid condensation agents, respectively. PS-LNPs had a defined hydrodynamic diameter and ζ-potential. Cellular uptake, transfection potency, and transfection efficiency of PS-LNPs were evaluated *in vitro* (i.e., HuH-7 hepatic carcinoma cell line) as well as *in vivo* (i.e., zebrafish embryo). EGFP: enhanced green fluorescent protein transgene.
**Figure 2****. Cellular transfection of PS-LNPs in vitro.** Transfection efficiency (TE) and transfection potency (TP, expressed as normalized EGFP signal intensity) of HuH-7 cells treated with DODMA LNPs containing increasing amounts of incorporated PS at N/P 10. a) 0.28 µg pDNA mL-1. 48 hr incubation. b) 0.28 µg mRNA mL-1. 24 hr incubation. Values are means ± SD, n = 3. Level of significance compared to 0% PS-LNPs: *: p ≤ 0.05, **: p ≤ 0.01, ***: p ≤ 0.001; Black stars: TE. Green stars: normalized EGFP signal intensity. c) Representative CLSM images of untreated control HuH-7 cells and cells treated with DODMA LNPs with 0%, 2.5%, and 5% incorporated PS. 0.28 µg nucleic acid mL-1. Left row: pDNA and 48 hr incubations. Right row: mRNA and 24 hr incubations. Green signal: EGFP. Cyan signal: Hoechst 33342 nuclear stain. Scale bar: 50 µm.
**Figure 3****: Cellular uptake of PS-LNPs.** HuH-7 cells were incubated with fluorescently labeled PS-LNPs (0 to 5% PS). Quantitation of cellular uptake of PS-LNPs condensing fluorescently labeled a) Cy5-pDNA or b) Cy5-mRNA at a final nucleic acid concentration of 0.034 µg mL-1 by flow cytometry (N/P 10, 4 hr incubation). Values are means ± SD, n = 3. Level of significance compared to 0% PS-LNPs: *: p ≤ 0.05, **: p ≤ 0.01, ***: p ≤ 0.001; Black stars: Cy5+ cells. Blue stars: normalized Cy5 signal intensity. c) Representative CLSM images of Dil-PS-LNP tracking analysis (N/P 10, 0.28 µg pDNA mL-1). Particle tracks are heat-mapped from blue (5 min) to red (30 min). Scale Bar: 10 µm.
**Figure** 4: *In vivo* evaluation of PS-LNP uptake and transfection in the zebrafish embryo (ZFE). Transfection efficiency (TE), transfection potency (TP), and biodistribution of LNPs were studied using ZFE. a) Schematic representation of the experimental procedure. After intravenous injection of LNPs, EGFP signal were visualized by CLSM within 13 hpi (for mRNA) to 42 hpi (for pDNA). Red: area of interest. b) Representative CLSM images of ZFE injected with PS-LNPs condensing either EGFP coding pDNA or mRNA. Color-coding according to EGFP signal intensity. Cyan: TE-fold difference as compared to 0% PS formulation; Yellow: TP-fold difference as compared to 0% PS formulation.. Scale Bar: 100 µm. c) Representative 3D-rendered image of a ZFE Tg(kdrl:EGFP) injected with Dil-labeled 2.5% PS-LNPs condensing pDNA. ZFE were analyzed 4 hpi. Green signal: ZFE vasculature, Red signal: Dil PS-LNPs associated with ZFE vasculature, Blue signal: Dil PS-LNPs not associated with ZFE vasculature. Scale Bar: 50 µm.

**Table 1: Physico-chemical properties of selected formulations as a function of the nucleic acid condensing lipids.** LNPs were prepared by bulk mixing and contained DOPC, Cholesterol, DOTAP or DODMA nucleic acid condensing lipids, nucleic acids at different N/P ratios, and different amounts of incorporated PS. pDNA: plasmid DNA, mRNA: 5' capped messenger RNA. Values are means ± SD, n ≥ 3.
**Table 2: Physico-chemical properties of selected formulations as a function of PS and N/P.** DODMA containing LNPs were prepared by microfluidics using the lipids DOPC, Cholesterol, DMPE-PEG2000, and different amounts of incorporated PS. pDNA: plasmid DNA, mRNA: 5' capped messenger RNA. Values are means ± SD, n = 3

### Examples

### Example 1

For Bulk Mixing: The lipid solutions (DODMA/DOTAP, Cholesterol, DOPC and PS at molar ratios of 50:40:10:0, 50:40:9.5:0.5, 50:40:9:1, 50:40:7.5:2.5, 50:40:5:5 and 50:30:20:10) were solved in CHCl₃ and dried on a Rotovap for 1 hour, rehydrated with 20 mM acetate / 5 % glucose buffer resulting in a liposome solution with a total lipid concentration of 0.8 mM. Liposomes were extruded 13 times by an Avanti Mini Extruder containing a Whatman nucleopore polycarbonate membrane with a pore size of 100 nm. For LNP formation, liposome solutions were diluted in 20 mM acetate / 5 % glucose buffer, the same was done with either mcDNA or mRNA to obtain the desired N/P ratio. The liposome-buffer solution was added to the nucleic acid-buffer solution and incubated at 22 °C for 30 minutes at 300 rpm. Physico-chemical properties of the resulting formulations are described in **Table 1.**

### Example 2

For Microfluidic Manufacturing: For the microfluidic (MF) LNPs, the lipid solutions (DODMA, Cholesterol, DOPC, PS and DMPE-PEG2000 at molar ratios of 50:39:10:0:1, 50:39:7.5:2.5:1 and 50:39:5:5:1) solved in CHCl₃ are dried under N₂ flow. The dry film was rehydrated with hot methanol (10 %) and ethanol (90 %) to obtain liposomes with the desired lipid concentration. The liposome solution and the nucleic acid-solution solved in 20 mM acetate / 5 % glucose buffer were diluted to obtain the desired N/P ratio. The samples were kept warm until used and were prepared at a total flow rate of 20 mL/min with an ethanol:H₂O phase ratio of 1:3 using two 3 mL syringes using a NanoAssemblr (Precision Nanosystems). Dialysis membranes (12'000 - 14'000 MWCO) were cut into 2-3 cm pieces and were incubated at 37 °C in H₂O under stirring for 3 hours. LNPs were dialyzed overnight against 1 mM HEPES / 0.9 % NaCl at pH 7.4. Physico-chemical properties of the resulting formulations are described in **Table 2.**

### Example 3

Different helper lipid compositions (PS, DOPC (1,2-dioleoyl-sn-glycerol-3-phosphocholine) and Cholesterol) were screened while using a constant amount (50 mol%) of either a cationic or ionizable lipid. Compositions can be summarized as follows: 0% PS: 50 mol% DOTAP/DODMA, 30 mol% Cholesterol, 10 mol% DOPC; 0.5% PS: 50 mol% DOTAP/DODMA, 30 mol% Cholesterol, 9.5 mol% DOPC, 0.5 mol% PS; 1% PS: 50 mol% DOTAP/DODMA, 30 mol% Cholesterol, 9 mol% DOPC, 1 mol% PS; 2.5% PS: 50 mol% DOTAP/DODMA, 30 mol% Cholesterol, 7.5 mol% DOPC, 2.5 mol% PS; 5% PS: 50 mol% DOTAP/DODMA, 30 mol% Cholesterol, 5 mol% DOPC, 5 mol% PS; 10% PS: 50 mol% DOTAP/DODMA, 20 mol% Cholesterol, 10 mol% DOPC, 10 mol% PS.These lipid formulations were used to condense either minicircle DNA (mcDNA) or messenger RNA (mRNA) coding for eGFP. Nitrogen to phosphate (N/P) ratios of 6 for DOTAP and 10 for DODMA formulations were investigated to achieve a high nucleic acid condensation while avoiding a possible excess of non-complexed LNPs. In this way a total of 36 formulations were screened. A hydrodynamic diameter (DH) between 126 nm ± 1 nm and 170 nm ± 3 nm with a polydispersity index (PDI) ranging between 0.1 and 0.16 was determined for DODMA LNPs whereas DOTAP LNPs resulted in a DH between 120 nm ± 1 nm and 150 nm ± 1 nm with a PDI between 0.04 and 0.13. No influence of PS integration was observed regarding size, PDI, and aggregation behavior. When incubated with fetal calf serum a slight increase in size distribution was observed, due to the previously described interaction of non-PEG LNPs with serum proteins but no aggregation of particles (> 1 µm), which could sediment and influence in vitro readout was observed. Physico-chemical properties of the resulting formulations are described in **Tables 1 and 2.**

### Example 4

ζ-potential measurements were performed at pH 4 (pH < pKₐ of the ionizable lipid) and at physiological pH 7.4 to follow the surface charge behavior of LNPs. At pH 7.4, a significant reduction in surface charge was observed for DODMA LNPs supplemented with PS. The pKₐ of the carboxyl and amino groups on the serine head group (3.6 and 9.1 respectively) gives PS molecules a net charge of -1 at physiological pH. In presence of PS, DODMA LNPs condensing pDNA at N/P 10 a reduction from 21 ± 1 mV for 0% PS to -4 ± 1 mV for 5% PS was observed. DOTAP is characterized by a tertiary amine and due to its non-ionizable nature holds a higher charge density and therefore DOTAP The ζ-potential of DOTAP LNPs with incorporated PS also decreased up to 30 mV. The resulting ζ-potentials for DOTAP PS-LNPs were still highly positive.At pH 4, regardless of the type of lipid, the ζ-potential was positive and approximately 30 mV. Physico-chemical properties of the resulting formulations are described in **Tables 1 and 2.**

### Example 5:

PS in LNPs can enhance the potency to transfect cells as shown by in cell culture. HuH-7cells were obtained from American Type Culture Collection and cultured in 55 cm² dishes at various concentrations at 37 °C under 5 % CO₂ and saturated humidity in in Dulbecco's modified Eagle's medium (DMEM) high glucose (4500 mg/L) supplemented with 10 % fetal calf serum (FCS) and 1 % Penicillin-Streptomycin (PS). Prepared eGFP encoding mcDNA LNP formulations were added to cells at 0.28 µg/mL and 0.83 µg/mL. After 48 hours, cells were trypsinized, resuspended in an appropriate buffer, and analyzed by flow cytometry. Two parameters were used as a read out: first, the transfection potency (TP, i.e., mean eGFP signal intensity expressed as single-cell relative fluorescence units), and second the transfection efficiency (TE) reflecting on the amount of eGFP positive cells.These experiments revealed a strong effect of incorporated PS on the transfection ability of LNPs formulated with DODMA as well DOTAP (Figure 2). These results can be summarized as follows: The TP of LNPs can be enhanced considerably using defined amounts of PS. Optimal results were obtained using 1 to 5% PS. Lower PS concentrations had no effect. Excessive concentrations of PS (i.e. PS content > 5%) had no or even detrimental effects as observed by a sharp decrease in TE for pDNA containing PS-LNPs. This can be attributed to the negative charge of PS interfering with pDNA condensation. It is important to note that incorporation of PS into LNPs had no effect on cell viability as determined by the MTS assay.

### Example 6

Since PS promotes receptor interactions of viral and exosomal particles, we investigated if the increased TP of PS-LNPs is a consequence of enhanced cellular uptake. Therefore, PS-LNPs condensing either fluorescently labeled Cy5-pDNA or Cy5-mRNA were incubated with HuH-7 cells at a final nucleic acid concentration of 0.034 µg mL-1 and were analyzed by flow cytometry and confocal microscopy **(****Figure 3****).** For pDNA LNPs 4 hr post-incubation, only slight changes regarding the amount of Cy5+ cells were observed (> 10%). Hoewver, an almost 1.6- respectively 1.3-fold increased Cy5 signal intensity was observed for formulations with 2.5% PS and 5% PS as compared to the 0% PS formulation. A similar trend was observed using mRNA PS-LNPs. Again, Cy5+ cells could be increased by 13% using 2.5% PS-LNPs. Furthermore, Cy5 signal intensity increased 1.6-fold for 2.5% PS formulation.

### Example 7

To elucidate mechanisms of cellular uptake and intracellular processing in the presence and absence of PS, we conducted a series of cellular uptake inhibition experiments. An arbitrary threshold of at least 50% inhibition was considered to be significant. Uptake of PS-LNPs (in contrast to LNPs) was specifically inhibited by Annexin V (which binds to PS and therefore blocks the uptake of apoptotic cells) and by Chlorpromazine (a known clathrin-mediated endocytosis inhibitor). Uptake of both PS-LNPs and LNPs was specifically inhibited by Colchicine (inhibitor of tubulin polymerization), Dynasore (inhibition of clathrin-mediated endocytosis), and Poly I:C (a synthetic single-stranded RNA that can competitively bind to the scavenger receptor SR-A). Remarkably, Poly I:C inhibited cellular uptake of LNPs by 50% but almost completely blocked the uptake of PS-LNPs.

### Example 8

The increased ability of PS-LNP to transfect cells observed in vitro was verified in vivo using the developing zebrafish embryo (ZFE) as an in vivo vertebrate screening model **(****Figure 4****).** Formulations were injected intravenously into the Duct of Cuvier of ZFE 36 hours post fertilization. In these experiments (and in contrast to the in vitro experiments), PS-LNPs were modified with PEGylated phospholipids (i.e., DMPE-PEG2000). This modification is necessary to suppress opsonization and agglomeration in presence of plasma protein. In addition, immune interactions can be suppressed such as inflammatory reactions or recognition of LNPs by cells of the mononuclear phagocyte system. Since PEGylation interferes with condensation of nucleic acids, these LNPs were prepared using a microfluidics protocol **(Table 2)** and not the bulk mixing **(Table 1).** In vivo experiments in ZFE confirmed our in vitro results **(****Figure 4****).** As expected and in agreement with previous in vitro results, free nucleic acids or LNPs devoid of PS show minimal activity 42 hours post injection (hpi). Addition of PS to pDNA-LNPs led to enhanced GFP expression in the tail region of the ZFE. Addition of PS to mRNA-LNPs resulted in a strong and widely distributed EGFP signal throughout the ZFE. A semi-quantitative analysis of the tail region of the ZFE revealed 3.1-fold versus 5.2-fold differences in TE between pDNA PS-LNP and mRNA PS-LNP treated ZFE. It can be concluded that PS enhances TE for both types of LNPs. No toxic effects were observed in ZFE after LNP administration.

**Table 1: Physico-chemical properties of selected formulations as a function of the nucleic acid condensing lipids**

| Formulation | % PS | N/P | pDNA | | | mRNA | | |
|---|---|---|---|---|---|---|---|---|
| | | | D_{H} [nm] ^{a)} | PDI ^{b)} | ζ-potential [mV] ^{c)} | D_{H} [nm] ^{a)} | PDI ^{b)} | ζ-potential [mV] ^{c)} |
| DOTAP | 0 | 6 | 138 ± 1 | 0.06 ± 0.01 | 55 ± 8 | 122 ± 1 | 0.09 ± 0.01 | 10 ± 3 |
| | 0.5 | 6 | 129 ± 2 | 0.05 ± 0.02 | 49 ± 3 | 120 ± 1 | 0.11 ± 0.01 | 13 ± 5 |
| | 1 | 6 | 122 ± 2 | 0.04 ± 0.03 | 47 ± 4 | 125 ± 0 | 0.06 ± 0.02 | 12 ± 1 |
| | 2.5 | 6 | 137 ± 3 | 0.06 ± 0.02 | 39 ± 9 | 122 ± 1 | 0.10 ± 0.01 | 7+ 1 |
| | 5 | 6 | 130 ± 1 | 0.07 ± 0.01 | 28 ± 2 | 134 ± 1 | 0.06 ± 0.01 | 3 ± 1 |
| | 10 | 6 | 150 ± 1 | 0.13 ± 0.01 | 35 ± 1 | 147 ± 1 | 0.10 ± 0.01 | 8+1 |
| DODMA | 0 | 10 | 126 ± 1 | 0.10 ± 0.02 | 21 ± 1 | 134 ± 0 | 0.11 ± 0.01 | 10 ± 1 |
| | 0.5 | 10 | 141 ± 3 | 0.15 ± 0.01 | -3 ± 2 | 136 ± 1 | 0.16 ± 0.02 | 5 ± 1 |
| | 1 | 10 | 150 ± 1 | 0.13 ± 0.01 | -17 ± 3 | 138 ± 1 | 0.14 ± 0.02 | -9 ± 0 |
| | 2.5 | 10 | 136 ± 2 | 0.13 ± 0.02 | -3 ± 0 | 128 ± 1 | 0.13 ± 0.01 | -13 ± 1 |
| | 5 | 10 | 170 ± 3 | 0.11 ± 0.01 | -4 ± 1 | 135 ± 1 | 0.13 ± 0.01 | -3 ± 1 |
| | 10 | 10 | 144 ± 2 | 0.11 ± 0.02 | -6 ± 2 | 132+ 1 | 0.13 ± 0.01 | -10 ± 1 |

| | | | | | | | | |
|---|---|---|---|---|---|---|---|---|
| ^{a)} hydrodynamic diameter, ^{b)} polydispersity index, ^{c)} ζ-potential at pH 7.4 | | | | | | | | |

**Table 2: Physico-chemical properties of selected formulations as a function of PS and N/P**

| | | pDNA | | | | mRNA | | | |
|---|---|---|---|---|---|---|---|---|---|
| % PS | N/P | D_{H} [nm] ^{a)} | PDI ^{b)} | ζ-potential [mV] ^{c)} | EE [%] ^{d)} | D_{H}[nm] ^{a)} | PDI^{b)} | ζ-potential [mV] ^{c)} | EE [%] ^{d)} |
| 0 | 6 | 114 ± 1 | 0.13 ± 0.01 | -6 ± 0 | 87 ± 6 | 126 ± 2 | 0.15 ± 0.03 | -3 ± 1 | 65 ± 14 |
| | 10 | 101 ± 1 | 0.15 ± 0.01 | -7 ± 1 | 88 ± 2 | 108 ± 1 | 0.20 ± 0.02 | -6 ± 2 | 76 ± 7 |
| 2.5 | 6 | 111 ± 1 | 0.12 ± 0.01 | -5 ± 0 | 93 ± 7 | 100 ± 2 | 0.16 ± 0.03 | -12 ± 1 | 54 ± 11 |
| | 10 | 103 ± 1 | 0.10 ± 0.01 | -8 ± 0 | 100 ± 3 | 92 ± 1 | 0.14 ± 0.01 | -5 ± 1 | 68 ± 10 |
| 5 | 6 | 108 ± 1 | 0.14 ± 0.00 | -6 ± 1 | 93 ± 3 | 105 ± 1 | 0.11 ± 0.00 | -11 ± 1 | 74 ± 12 |
| | 10 | 102 ± 0 | 0.20 ± 0.00 | -6 ± 1 | 101 ± 2 | 93 ± 1 | 0.19 ± 0.00 | -5 ± 1 | 78 ± 6 |

| | | | | | | | | | |
|---|---|---|---|---|---|---|---|---|---|
| a) hydrodynamic diameter, ^{b)} polydispersity index, ^{c)} ζ-potential at pH 7.4, ^{d)} encapsulation efficiency | | | | | | | | | |

## Claims

1. A lipid nanoparticle (LNP) comprising at least one cationic matrix-forming lipid and 0.5 mol% to 10 mol% phosphatidylserine (PS).

2. The LNP of claim 1 additionally comprising a protein and/or a negatively charged nanoparticle.

3. A vector comprising the LNP of claim 1 or 2 and a nucleic acid.

4. The vector of claim 3, wherein the nucleic acid is condensed nucleic acid.

5. The vector of claim 3 or 4, wherein the vector has an N/P ratio between 1 and 10.5.

6. The LNP of any one of claims 1 or 2 or the vector of any one of claims 3 to 5, wherein the LNP additionally comprises at least one helper lipid.

7. The LNP of claim 6, or the vector of claim 6, wherein the helper lipid comprises a PEG-modified lipid, preferably accounting for 0.5% to 10% of the LNP mass.

8. The LNP of claim 6 or 7, or the vector of claim 6 or 7, wherein the helper lipid comprises DOPC and/or cholesterol.

9. A pharmaceutical composition comprising the LNP of any one of claims 1, 2 or 6 to 8, or the vector of any one of claims 3 to 8 and a pharmaceutical carrier.

10. The LNP, vector or pharmaceutical composition of any of the preceding claims for use in medicine.

11. The LNP of claims 1, 2, 6 to 8, the vector of claims 3 to 8 or the pharmaceutical composition of claim 9, for use in the treatment and/or prevention of a disease or disorder selected from the group of genetic disease or disorder, infectious disease or disorder, cancerous disease or disorder,
preferably wherein the genetic disease or disorder is sickle cell anemia or amyloidosis.

12. The LNP, vector or pharmaceutical composition for use of claim 11, wherein the infectious disease or disorder is a viral infection

13. A needle-free injector device comprising the pharmaceutical composition of claim 9, the LNP of any one of claims 1, 2 or 6 to 8, or the vector of any one of claims 3 to 8.

14. A method for producing a vector of any of the claims 3 to 8 comprising the steps of:
a) mixing a matrix-forming lipid, an ionizable lipid with 0.5 mol% to 10 mol% phosphatidylserine;
b) obtaining a lipid nanoparticle by facilitated self-assembly and by a size-dependent separation method of the mixture of step (a); and
c) obtaining the vector by loading the lipid nanoparticle with a nucleic acid.

15. A method for producing a vector of any one of the claims 3 to 8 comprising the steps of:
a) mixing a matrix-forming lipid, an ionizable lipid and 0.5 mol% to 10 mol% phosphatidylserine with a nucleic acid; and
b) obtaining the vector by facilitated self-assembly and by a size-dependent separation method of the mixture of step (a).
